# DEMANDE DE BREVET EUROPEEN

(11) **EP 1 022 018 A1**
(43) Date de publication de la demande: **26.07.2000**
(21) Numéro de dépôt: 00400040.2
(22) Date de dépôt: 10.01.2000
(51) Int. Cl.: A61K 7/48

(54) **Utilisation d'au moins un extrait d'au moins une bactérie filamenteuse non photosynthétique en tant qu'agent amincissant**

(30) Priorité: 15.01.1999 FR 9900405
(71) Demandeur: L'OREAL, 75008 Paris (FR)
(72) Inventeur: Liviero, Christelle, 75008 Paris (FR); Breton, Lionel, 78000 Versailles (FR); Allec, Josiane, Les Vergères du Val Constance, 06600 Antibes (FR); Martin, Richard, 37210 Rochecorbon (FR)
(74) Mandataire: Galup, Cédric Olivier Nicolas

(57) **Abrégé**

La présente invention concerne l'utilisation dans une composition d'au moins un extrait d'au moins une bactérie filamenteuse non photosynthétique en tant qu'agent amincissant.
Elle concerne en outre une composition cosmétique amincissante comprenant dans un milieu cosmétiquement acceptable au moins un extrait d'au moins une bactérie filamenteuse non photosynthétique et un procédé de traitement cosmétique destiné à favoriser l'amincissement ou l'affinement du corps et de la silhouette ou de certaines parties du corps et de la silhouette, caractérisé par le fait que l'on applique sur la peau une telle composition.

## Description

La présente invention concerne l'utilisation dans une composition d'au moins un extrait d'au moins une bactérie filamenteuse non photosynthétique pour diminuer et/ou lutter contre les rondeurs et/ou surcharges pondérales en vue d'obtenir un effet d'amincissement généralisé ou localisé du corps humain ou animal. Elle se rapporte aussi à un procédé d'amincissement du corps, consistant à appliquer par voie topique une composition comprenant au moins un extrait d'au moins une bactérie filamenteuse non photosynthétique.

Les rondeurs et/ou surcharges pondérales sont liées au dysfonctionnement de certaines cellules de l'hypoderme, appelées adipocytes, qui contiennent des quantités variables de graisses stockées sous la forme de triglycérides. Ces triglycérides sont synthétisés *in vivo* par les adipocytes eux-mêmes, selon des réactions de type enzymatique (lipogenèse), à partir des acides gras libres et du glucose contenus dans l'organisme et apportés à celui-ci par l'intermédiaire de certains aliments. La transformation du glucose conduit soit à la formation de glycérol, soit à la formation d'acides gras libres par l'intermédiaire d'une enzyme spécifique, l'acétylCoa-carboxylase. Or, parallèlement, les triglycérides ainsi formés, puis stockés, dans les cellules adipocytes peuvent également se redécomposer(lipolyse), toujours sous l'action d'enzymes spécifiques (triglycérides lipases) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogenèse.

Le métabolisme des acides gras est donc l'une des cibles privilégiées dans le contrôle de cette surcharge lipidique adipocytaire.
Ainsi, le blocage du transport du glucose à l'intérieur de l'adipocyte conduit également à une diminution de la synthèse de triglycérides (puisque le glucose est transformé en Acétyl Coa puis en Acides Gras, via l'activité de l'Acetyl Coa Carboxylase)
De la même façon, toute substance susceptible de bloquer l'entrée et/ou la sortie des acides gras libres de l'adipocyte doit conduire à une variation de la charge adipeuse.
En effet, le transport d'acides gras est sous le contrôle de transporteurs spécifiques, dont la protéine de transport des acides gras (cf. The Journal Of Biological Chemistry, vol 272, pp 28210-28217, 1997) et la modification de ces protéines conduit à une variation du taux en triglycérides de l'adipocyte.

Si, pour des raisons diverses (nourriture trop riche, inactivité, variation du métabolisme, vieillissement et autres), un déséquilibre substantiel s'installe dans l'organisme entre la lipogenèse (formation de triglycérides par réaction enzymatique entre des acides gras et le glycérol) et la lipolyse (décomposition enzymatique de triglycérides en acides gras et glycérol), c'est-à-dire plus précisément si les quantités de graisses formées par lipogenèse deviennent notablement et constamment supérieures à celles qui sont éliminées par lipolyse, il se produit alors dans les adipocytes une accumulation de triglycérides qui, si elle devient excessive, peut se traduire progressivement par une déformation de la peau provoquée par l'épaississement de l'hypoderme dans lequel se trouvent les adipocytes. La surface de la peau devient irrégulière ("peau d'orange") et de consistance plus ou moins flasque ou gélatineuse, donnant finalement à la silhouette un aspect général disgracieux pouvant évoluer entre la simple surcharge locale (lipodismorphie), en passant par l'embonpoint certain, et enfin la réelle obésité.

Or, compte tenu notamment du profond inconfort tant physique qu'esthétique, et parfois psychologique, qu'elle occasionne auprès des individus qui en sont atteints, en particulier chez les femmes, l'adiposité constitue de nos jours une affection de moins en moins bien supportée ou acceptée.

Des méthodes ont certes déjà été proposées en vue de traiter l'adiposité, mais parmi celles-ci, seules en fait les méthodes reposant sur des traitements chirurgicaux, tels que la liposuccion, permettent actuellement d'obtenir des résultats véritablement satisfaisants. Toutefois, de tels traitements présentent bien évidemment comme inconvénient majeur de nécessiter la mise en oeuvre sur le corps humain ou animal d'opérations invasives par nature délicates, non sans risques et souvent coûteuses.

De même dans l'art antérieur il a été proposé l'utilisation de composés dits amincissants comme par exemple des composés dits "stimulateur" de lipolyse, c'est-à-dire des substances qui, *in vivo*, permettent, directement ou indirectement, de stimuler l'activité de lipolyse dans les adipocytes. Parmi ceux-ci, on peut plus particulièrement citer les bases xanthiques (i.e. des dérivés de la xanthine), telles que la théophylline, la caféine, la théobromine et les 1-hydroxyalkylxanthines et leurs sels compatibles (voir notamment à ce sujet le document FR-A- 2 617 401), les dérivés de l'acide nicotinique tels que plus particulièrement le nicotinate d'alpha-tocophérol et le nicotinate d'hexyle (voir notamment à ce sujet le document EP-A- 371 844), les substances dites alpha-2 bloqueurs capables de bloquer les récepteurs alpha-2 à la surface des adipocytes comme par exemple le ginkgo biloba (voir notamment à ce sujet le document FR-A- 2 669 537), et enfin les facteurs de croissance (voir notamment à ce sujet le document FR-A- 2 671 487).

Il n'en demeure pas moins qu'il subsiste encore aujourd'hui un fort besoin quant à pouvoir disposer d'un procédé de traitement cosmétique et/ou thérapeutique "doux", de type non chirurgical, et permettant de lutter efficacement contre l'adiposité humaine ou animale, et ceci en vue notamment d'obtenir un effet général, ou au contraire localisé, d'amincissement et/ou d'affinement du corps ou de la silhouette.

La présente invention vise justement à la satisfaction d'un tel besoin.

La demanderesse a découvert qu'un extrait de bactéries filamenteuses non photosynthétiques répond aux caractéristiques d'un agent amincissant.

A la connaissance de la demanderesse il n'a jamais été envisagé ni suggéré dans l'art antérieur qu'un extrait d'au moins une bactérie filamenteuse non photosynthétique peut avoir une activité amincissante.

Par activité amincissante d'un composé, on entend l'activité d'un composé qui conduit à diminuer les rondeurs et/ou surcharges pondérales soit parce qu'il inhibe la lipogenèse, soit parce qu'il stimule la lipolyse soit encore parce qu'il possède les deux activités.

Ainsi, l'invention concerne l'utilisation dans une composition d'au moins un extrait d'au moins une bactérie filamenteuse non photosynthétique comme agent amincissant.

La présente invention trouve ainsi des applications particulièrement utiles et intéressantes dans le domaine des traitements cosmétiques visant à obtenir des effets locaux ou généralisés d'amincissement et/ou affinement de la peau ou de la silhouette (hanches, fesses, cuisses, ventre et autres).

L'un des grands avantages de la présente invention réside dans la possibilité de pouvoir procéder, chaque fois que nécessaire ou souhaitable, à des traitements "doux" très localisés et sélectifs grâce au mode d'application par voie topique.

La présente invention est bien entendu applicable au corps tant humain qu'animal.

Par extrait de bactéries filamenteuses non photosynthétiques, on entend aussi bien le surnageant de culture desdites bactéries, la biomasse brute obtenue par séparation après culture desdites bactéries ou encore les extraits de la biomasse, quel que soit leur degré de purification, obtenus par traitement de cette biomasse.

Préférentiellement selon l'invention, on utilise un extrait de bactéries filamenteuses non photosynthétiques enrichi en lipopolysaccharides (LPS). Par extrait enrichi en LPS, on entend un extrait qui, à partir de la biomasse brute obtenue par séparation après culture des bactéries, a subi au moins une étape permettant l'augmentation de la quantité de LPS qu'il contient par rapport à ce qu'il aurait contenu après une simple lyse des bactéries. En d'autres termes, cet extrait a subi au moins une étape de concentration en LPS. Préférentiellement selon l'invention, l'extrait peut contenir de 20% à 98% de LPS.

Un exemple de protocole de réalisation d'une telle préparation est donné par ailleurs dans le texte.

Les extraits de bactéries selon l'invention sont préparés à partir de bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.

Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

Préférentiellement, on utilise selon l'invention une souche de *Vitreoscilla filiformis*.

Pour préparer l'extrait selon l'invention, on peut cultiver lesdites bactéries selon les méthodes connues de l'homme du métier, puis séparer la biomasse obtenue, par exemple par filtration, centrifugation, coagulation et/ou lyophilisation.
On peut notamment préparer les extraits utilisables selon l'invention, selon le procédé décrit par la demanderesse dans la demande de brevet WO-A-93/00741.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.
La fraction surnageante de cette biomasse autoclavée peut également être filtrée dans un récipient stérile pour éliminer les particules en suspension.

Pour obtenir l'extrait enrichi en LPS, la biomasse obtenue par centrifugation est resuspendue dans un tampon de solubilisation tel que décrit par Zanen O.G. & H.C. Zanen, (FEMS Microbiol. Letters, 1988, 50, 85-88), puis de nouveau centrifuger. Le surnageant est alors traité avec une enzyme protéolytique et l'enrichissement en LPS se fait par précipitation successive en milieu alcoolique. La préparation peut alors subir une épuration par dialyse.

L'extrait peut constituer à lui seul le principe actif des compositions de l'invention.

La quantité d'extrait de bactéries filamenteuses non photosynthétiques utilisable selon l'invention dépend bien évidemment de l'effet recherché et doit être en une quantité efficace pour favoriser l'amincissement.

Selon l'invention, l'extrait bactérien peut être utilisé en une quantité représentant de 0,1% à 80% du poids total de la composition et préférentiellement en une quantité représentant de 0,2% à 40% du poids total de la composition.

La composition de l'invention peut être à usage cosmétique ou pharmaceutique. Préférentiellement, la composition de l'invention est à usage cosmétique.

L'extrait bactérien peut être utilisé dans une composition qui doit être ingérée, injectée ou appliquée sur la peau (sur toute zone cutanée du corps). Préférentiellement, la composition de l'invention est appliquée sur la peau

Selon le mode d'administration, cette composition peut se présenter sous toutes les formes galéniques normalement utilisées.

Pour une application topique sur la peau, la composition peut avoir la forme notamment d'une solution ou suspension aqueuse, alcoolique ou hydroalcoolique ou d'une suspension huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle du type crème H/E ou E/H ou d'un gel aqueux ou anhydres, d'un onguent, d'une poudre libre ou compactée à utiliser telle quelle ou à incorporer dans un excipient, ou encore de microcapsules ou microparticules, ou de dispersions vésiculaires de type ionique et/ou non ionique. Ces compositions sont préparées selon les méthodes usuelles.

On peut également envisager une composition sous la forme d'une mousse ou encore sous forme de compositions pour aérosol comprenant également un agent propulseur sous pression.

Pour l'injection, la composition peut se présenter sous forme de lotion aqueuse, de suspension huileuse ou sous forme de sérum. Pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes pour le corps, des laits corporels, des lotions, des gels ou des mousses.

Les compositions selon l'invention peuvent également consister en des produits de nettoyage (savons, pains, crèmes ou gels moussants).

Lorsque la composition est une émulsion, la proportion de la phase grasse peut aller de 2 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,1 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthyléne.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention, on peut, entre autres, associer au moins un extrait d'au moins une bactérie filamenteuse non photosynthétique à d'autres composés choisis parmi :
- les hormones végétales ;
- les agents antibactériens tels que les macrolides, les pyranosides et les tétracyclines, et notamment l'érythromycine ;
- les agents antagonistes de calcium, comme le vérapamil et le Diltiazem ;
- des capteurs de radicaux OH, tels que le diméthylsulfoxyde ;
- des extraits de végétaux tels que ceux d'Iridacées ou de soja, extraits pouvant alors contenir ou non des isoflavones ;
- des extraits de micro-organismes autres que les extraits de bactéries filamenteuses non photosynthétiques .
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les agents modulant l'adhésion bactérienne sur la peau et /ou les muqueuses tels que le miel, notamment le mile d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétindïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Bien entendu, il est possible d'associer à l'extrait de bactéries filamenteuses non photosynthétiques un composé connu pour présenter des propriétés amincissantes choisi parmi les composés dits "stimulateur" de lipolyse comme par exemple les bases xanthiques telles que la théophylline, la caféine, la théobromine et les 1-hydroxyalkylxanthines et leurs sels compatibles, les dérivés de l'acide nicotinique tels que plus particulièrement le nicotinate d'alpha-tocophérol et le nicotinate d'hexyle, les substances dites α-2 bloqueurs capables de bloquer les récepteurs α-2 à la surface des adipocytes comme par exemple le ginkgo biloba, les facteurs de croissance, les bloqueurs du transport du glucose tel que la rutine, les agonistes β-adrénergiques comme par exemple I'isoprotérénol, ou encore les antagonistes du neuropeptide Y (NPY) capable de bloquer les récepteurs NPY à la surface des adipocytes comme par exemple des extraits d'origine végétale ou bactérienne.

Ainsi, selon un mode particulier, l'invention concerne une composition comprenant au moins un extrait d'au moins une bactérie filamenteuse non photosynthétique et un composé connu pour présenter des propriétés amincissantes choisi parmi les inhibiteurs de phosphodiestérase comme par exemple les bases xanthiques telles que la théophylline, la caféine, la théobromine et les 1-hydroxyalkylxanthines et leurs sels compatibles, les dérivés de l'acide nicotinique tels que plus particulièrement le nicotinate d'alpha-tocophérol et le nicotinate d'hexyle, les substances dites α-2 bloqueurs capables de bloquer les récepteurs α-2 à la surface des adipocytes comme par exemple le ginkgo biloba, les bloqueurs du transport du glucose tel que la rutine, les agonistes β-adrénergiques comme par exemple l'isoprotérénol, ou encore les antagonistes du neuropeptide Y (NPY) capable de bloquer les récepteurs NPY à la surface des adipocytes comme par exemple des extraits d'origine végétale ou bactérienne, les agents modifiant le transport des acides gras comme par exemple les lipopolysaccharides..

La présente invention a en outre pour objet un procédé de traitement cosmétique en vue de favoriser l'amincissement ou l'affinement du corps et de la silhouette ou de certaines parties du corps et de la silhouette, caractérisé par le fait que l'on applique sur la peau une composition cosmétique comprenant au moins un extrait de bactéries filamenteuses non photosynthétiques

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits sur la peau.

Les exemples et compositions suivants illustrent l'invention sans la limiter aucunement. Dans les compositions, les proportions indiquées sont des pourcentages en poids

### Exemple 1 : Préparation d'un extrait de Vitreoscilla filiformis :

La souche de *Vitreoscilla filiformis* (ATCC 15551) est mise en culture selon le procédé décrit dans la demande de brevet WO-A-93-00741. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % V/V dans du milieu neuf toutes les 48 heures jusqu'à l'obtention d'une culture stable. Un Erlenmeyer de 1 litre contenant 200 ml de milieu neuf est alors ensemencé avec 4 ml de la culture précédente.

La culture en Erlenmeyer s'effectue à 26° C sur une table de culture agitée à 100 tours/minutes. Le pied de cuve ainsi obtenu sert d'inoculum à un fermenteur de 10 l. La croissance s'effectue à 26° C, pH 7,100 tours/minute et pO₂ ≥ 15 %. Après 48 heures de croissance, la biomasse est transférée dans un fermenteur de 600 litres utiles, pour être cultivée dans les mêmes conditions. Après 48 H de croissance on récolte les cellules. La biomasse est alors concentrée 50 fois environ par centrifugation.
Le concentré est autoclavé à 121° C durant 40 minutes. Après refroidissement, 2 phases apparaissent. La phase liquide surnageante est alors filtrée à 0,22 µm pour éliminer les particules. Cet extrait est utilisable en l'état (forme aqueuse) ou peut être lyophilisé suivant les techniques classiques (forme lyophilisée).

### Exemple 2 : Préparation d'un extrait enrichi en LPS de Vitreoscilla filiformis :

6x70 ml du concentré autoclavé de l'exemple 1 sont centrifugés à 100.000 G à 4°C pendant 1h30′
Les culots récupérés sont resuspendus dans 150 ml de tampon de solubilisation [Tris/HCl 0,625 M pH 6,8 contenant 4% (w/v) de SDS (Sodium Dodecyl Sulfate), 10% (v/v) de 2 mercaptoéthanol et 20% (v/v) de glycérol. (Zanen O.G. & H.C. Zanen, FEMS Microbiol. Letters, 1988, 50, 85-88)] et maintenus à 100°C pendant 1 heure, puis de nouveau centrifugés à 10.000 G à 4°C pendant 30 minutes.
On traite 100 ml du surnageant ainsi obtenu par 60 mg de protéinase K (SIGMA) à 60°C pendant 1 heure puis on ajoute 200 ml de MgCl₂ à 0,375 M dans de l'éthanol à 90% et on laisse à -20°C pendant 1 Nuit. Après centrifugation (10000 G, 4°C, 20′), le précipité recueilli est de nouveau soumis à deux cycles de précipitation en milieu alcoolique selon un protocole identique. Après une nouvelle centrifugation, le culot est repris dans quelques millilitres d'eau distillée. La préparation est ensuite épurée par 3 cycles de dialyse, une fois contre un tampon phosphate de sodium à pH 7 durant 24 heures et 2 fois contre de l'eau distillée durant 48h.
Après lyophilisation, on obtient ainsi 94 mg de LPS de Vitreoscilla filiformis bruts secs.

### Exemple 3 : Activité pharmacologique de l'extrait enrichi en LPS de l'exemple 2 :

Mesure de la quantité d'acide gras non estérifiés présents dans une culture d'adipocytes en présence de l'extrait enrichi en LPS de l'exemple 2. La quantité d'acide gras non estérifié présent dans la culture représente l'hydrolyse des triglycérides contenus dans les adipocytes, donc représente l'effet lipolytique du composé testé.

### Modèle Expérimental

### Cellules :

Les adipocytes ont été isolés d'une plastie de cuisse de donneur de sexe féminin de 66 ans.
On incube les fragments de tissus adipeux pendant 30 minutes à 37°C en présence de collagénase (Sigma C6885) puis on les reprends dans du milieu d'essai à raison de 10 ml de milieu par gramme de tissu adipeux.
Le milieu d'essai est composé de :

| | |
|---|---|
| bicarbonate ( life technologies 25080060) | 2.9% |
| pénicilline/ Streptomycine ( life technologies 15070022) | 0.5% |
| glutamine ( life technologies 25030024) | 1% |
| milieu MEM (Seromed 55034) contenant 0.5%(p/v) de sérumalbumine bovine (Sigma A7511) | Qsp 100% |

### Produits à tester et références :

Les deux références, la théophylline (inhibiteur de la phosphodiestérase) et l'isoprotèrénol (agoniste des récepteurs β1, β2 et β3) sont testés aux concentrations de 1 mM et 1 µM respectivement. Le LPS est testé aux concentrations de 100 µg/ml; 30µg/ml; 10 µg/ml et 3 µg/ml.
Ces différents actifs sont incorporés au milieu contenant les adipocytes et on dose les acides gras libres non estérifiés.

### Dosage :

On ajoute 60 µl de composé à tester à 540 µl de suspension d'adipocytes et les mélanges ont été incubés dans un bain marie à 37°C, sous agitation, pendant 2 heures.

Les acides gras non estérifiés sont dosés dans des fractions de 30 µl de milieu après décantation de 5 minutes.

Le dosage est réalisé à l'aide d'un kit NEFA-C (WACO 994-75409) en densitométrie.

Les données brutes de comptage sont transférées et traitées sous le logiciel PRISM (Graph pad Software) ; les comparaisons intergroupes ont été réalisées par analyse de variance (ANOVA) à l'aide du test de comparaison multiple de Dunnett.

### Résultats :

| Traitement | AGNE* (mM) | % AGNE* | p |
|---|---|---|---|
| non traité | 0.136 | - | - |
| théophylline 10-3M | 0.760 | +460 | <0.01 |
| isoprotérénol 10-6M | 0.785 | +478 | <0.01 |
| LPS 100µg/ml | 0.289 | +113 | <0.01 |
| LPS 30µg/ml | 0.204 | +51 | >0.05 |
| LPS 10µg/ml | 0.112 | - | >0.05 |
| LPS 3 µg/ml | 0.088 | - | >0.05 |

| | | | |
|---|---|---|---|
| * : AGNE : Acides Gras Non Estérifiés | | | |

Les références stimulent la lipolyse.

L'extrait enrichi en LPS stimule de façon dose-dépendante la libération des acides gras non estérifiés aux concentrations de 100 µg/ml et 30 µg/ ml avec un effet significatif à 100 µg/ml.
Aucun effet n'est détecté aux concentrations inférieures (10 µg/ml et 3 µg/ml).

Ces résultats mettent en évidence l'activité lipolytique de l'extrait de LPS isolée à partir de Vitreoscilla filiformis.

### Exemple 4 :

Exemples de formulations illustrant l'invention et particulièrement les compositions selon l'invention associant au moins un extrait d'au moins une bactérie filamenteuse non photosynthétique. Ces compositions ont été obtenues par simple mélange des différents composants.

### Composition 1 : Lotion amincissante

| | |
|---|---|
| Extrait de l'exemple 2 | 0,50 |
| Antioxydant | 0,05 |
| Ethanol | 40,00 |
| Conservateur | 0,30 |
| Eau | qsp 100 % |

### Composition 2 : Gel amincissant

| | |
|---|---|
| Extrait de l'exemple 2 | 0,50 |
| Hydroxypropylcellulose (Klucel H® vendu par la société Hercules) | 1,00 |
| Antioxydant | 0,05 |
| Ethanol | 40,00 |
| Conservateur | 0,30 |
| Eau | qsp 100 % |

### Composition 3 : Crème amincissante (émulsion huile dans eau)

| | |
|---|---|
| Extrait de l'exemple 2 | 2,00 |
| Stéarate de glycérol | 2,00 |
| Polysorbate 60 (Tween 60® vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Composition 4 : Crème amincissante (émulsion huile/eau)

| | |
|---|---|
| Extrait de l'exemple 2 | 0,50 |
| Stéarate de glycérol | 2,00 |
| Polysorbate 60 (Tween 60® vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Composition 5 : Gel amincissant

| | |
|---|---|
| Extrait de l'exemple 2 | 0,20 |
| Hydroxypropylcellulose (Klucel H® vendu par la société Hercules) | 1,00 |
| Antioxydant | 0,05 |
| Chlorhydrate de lidocaïne | 2,00 |
| Ethanol | 40,00 |
| Conservateur | 0,30 |
| Eau | qsp 100 % |

### Composition 6 : Crème amincissante (émulsion huile-dans-eau)

| | |
|---|---|
| Extrait de l'exemple 2 | 0,75 |
| Stéarate de glycérol | 2,00 |
| Polysorbate 60 (Tween 60® vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

### Composition 7 : Gel amincissant

| | |
|---|---|
| Extrait de l'exemple 2 | 0,50 |
| Acide tout trans rétinoïque | 0,05 |
| Hydroxypropylcellulose (Klucel H®) | 1,00 |
| Antioxydant | 0,05 |
| Ethanol | 40,00 |
| Conservateur | 0,30 |
| Eau | qsp 100 % |

### Composition 8 : Lotion amincissante

| | |
|---|---|
| Extrait de l'exemple 2 | 0,50 |
| Acide glycolique | 50,00 |
| Hydroxypropylcellulose (Klucel H®) | 0,05 |
| NaOH | qsp pH =2,80 |
| Ethanol | qsp 100 % |
| Conservateur | 0,30 |

## Revendications

1. Utilisation dans une composition d'au moins un extrait d'au moins une bactérie filamenteuse non photosynthétique comme agent amincissant.

2. Utilisation selon la revendication 1, caractérisée en ce que l'extrait est enrichi en lipopolysaccharides.

3. Utilisation selon l'une quelconque des revendications 1 ou 2, caractérisée par le fait que ladite bactérie appartient à l'ordre des Beggiatoales.

4. Utilisation selon la revendication 3, caractérisée en ce que ladite bactérie appartient au genre Beggiatoa, Vitreoscilla, Flexithrix ou Leucothrix.

5. Utilisation selon la revendication 4, caractérisée en ce que ladite bactérie est choisie parmi des souches de *Vitreoscilla filiformis*.

6. Utilisation selon l'une quelconque des revendications 1 à 5, caractérisée en ce que ledit extrait bactérien est utilisé en une quantité représentant de 0,1% à 80% du poids total de la composition.

7. Utilisation selon la revendication 6, caractérisée en ce que ledit extrait bactérien est utilisé en une quantité représentant de 0,2% à 40% du poids total de la composition.

8. Composition, caractérisée en ce qu'elle comprend au moins un extrait d'au moins une bactérie filamenteuse non photosynthétique et un composé connu pour présenter des propriétés amincissantes choisi parmi les inhibiteurs de phosphodiestérase, les dérivés de l'acide nicotinique, les substances dites α-2 bloqueurs capables de bloquer les récepteurs α-2 à la surface des adipocytes, les bloqueurs du transport du glucose, les agonistes β-adrénergiques, les antagonistes du neuropeptide Y (NPY) capable de bloquer les récepteurs NPY à la surface des adipocytes comme par exemple des extraits d'origine végétale ou bactérienne, les agents modifiant le transport des acides gras.

9. Composition selon la revendication 8, caractérisée par le fait que ladite bactérie est telle que définie dans l'une quelconque des revendications 2 à 7.

10. Procédé de traitement cosmétique en vue de favoriser l'amincissement ou l'affinement du corps et de la silhouette ou de certaines parties du corps et de la silhouette, caractérisé par le fait que l'on applique sur la peau une composition comprenant dans un milieu cosmétiquement acceptable au moins un extrait d'au moins une bactérie filamenteuse non photosynthétique.
